# EUROPÄISCHE PATENTANMELDUNG

(11) **EP 0 667 162 A1**
(43) Veröffentlichungstag der Anmeldung: **16.08.1995**
(21) Anmeldenummer: 94102097.6
(22) Anmeldetag: 11.02.1994
(51) Int. Cl.: A61K 35/78

(54) **Pflanzliches Heilmittel und Verfahren zu dessen Herstellung**

(71) Anmelder: Brunner, Rudolf, CH-8307 Effretikon (CH)
(72) Erfinder: Brunner, Rudolf, CH-8307 Effretikon (CH)
(74) Vertreter: Blum, Rudolf Emil Ernst

(57) **Zusammenfassung**

Es werden pflanzliche Heilmittel auf Basis von Pflanzenextrakten beschrieben, insbesondere auf Basis von Aronstab, Bärlauch und Tollkirsche, sowie ein Extraktionsverfahren zu dessen Herstellung. Diese Heilmittel zeigen bei vielen Krankheiten wie Entzündungen, Virusinfektionen, Geschwulsten und Ekzemen schmerzstillende, fiebersenkende und heilende Wirkung.

## Beschreibung

Die vorliegende Erfindung betrifft ein natürliches Heilmittel auf Basis von Pflanzenextrakten und dessen Herstellung.

Die Heilkräfte vieler Pflanzen sind bekannt und viele der heute vertriebenen Arzneimittel basieren auf Heilpflanzen resp. deren Inhaltsstoffen.

Bisher ist aber noch kein nebenwirkungsarmes Heilmittel bekannt, das ausschliesslich auf Pflanzenextrakten basiert und ein breites Anwendungsspektrum aufweist, welches sowohl Entzündungen als auch Virusinfektionen, Geschwulste und Ekzeme einschliesst.

Ziel dieser Erfindung ist es deshalb, ein solches Heilmittel sowie ein Verfahren zu dessen Herstellung bereitzustellen.

Das erfindungsgemässe natürliche Heilmittel enthält Extrakte von Pflanzen der Familien der Solanaceae (Nachtschattengewächse), Araceae (Aronstabgewächse) und Liliaceae (Liliengewächse) insbesondere der Gattung Allium.

Speziell geeignete Pflanzen sind die in Mitteleuropa heimischen Pflanzen Tollkirsche, Aronakraut (Aronstab, Arum maculatum) und Bärlauch.

Die Extrakte sind gewöhnlich alkoholischer Natur, wobei Ethanol aus physiologischen Gründen stark bevorzugt ist.

Bereits die Einzelextrakte dieser Pflanzen weisen gewisse Heilwirkungen auf, so tragen die Extrakte aus den Blättern (und Stielen) des Bärlauchs resp. des Aronakrauts beispielsweise zur Verbesserung und Reinigung des Blutes bei, während das Extrakt aus der Frucht der Tollkirsche bei z.B. Magenkrebs gute Wirkung gezeigt hat.

Ausserdem wurde gefunden, dass sich die Wirkung dieser Pflanzenextrakte durch deren Kombination verstärken lässt, wodurch sich auch der Anwendungsbereich des Kombinationspräparates vergrössern lässt.

Die Zusammensetzung des Kombinationspräparates kann variieren. Für eine gute Anwendungsbreite hat sich eine Zusammensetzung bewährt, die auf identisch hergestellten Extrakten basiert, welche im nachfolgend aufgeführten Verhältnis gemischt wurden, wobei die Komponente zusammen 100 % ergeben.
65-75 % Extrakt von Aronakraut (Blätter und Stiele)
15-25 % Extrakt von Bärlauch (Blätter und Stiele) 5-15 % Extrakt von Tollkirsche (Frucht).

Das erfindungsgemässe Kombinationspräparat der obengenannten Zusammensetzung eignet sich zur Behandlung von Entzündungen, Geschwuren, z.B. diversen Krebsarten, Ekzemen, Diabetes, Greisenbrand und Viruserkrankungen wie Gürtelrose (Zoster) und Aids. Das Heilmittel wirkt schmerzstillend, fiebersenkend und heilend. Es eignet sich sowohl für die topische als auch für die orale Anwendung.

Die erfindungsgemässen Präparate werden hergestellt, indem die Wirkstoffe der Pflanzen mit Alkohol, bevorzugt Ethanol extrahiert werden, wobei das verwendete pflanzliche Material der Liliaceae, insbesondere des Bärlauchs und der Araceae, insbesondere des Aronakrauts (Aronstabs), das über dem Boden stehende Kraut (Blätter sowie Stiele) ist, bei den Solanaceae, insbesondere der Tollkirsche, die Früchte.

Es wurde gefunden, dass für die Extraktion mindestens 40 %iger Alkohol verwendet werden muss, bevorzugt ist dieser allerdings 70 %ig.

Eine einfache und schonende Art der Extraktion ist einfaches Stehenlassen der mit Alkohol bedeckten Kräuter bei Umgbungstemperatur, in einem geschlossenen Gefäss.

Gute Heilwirkung wurde bei Präparaten gefunden, die aus Extrakten hergestellt wurden, welche aus 3-4 kg Kräutern resp. Früchten pro 20-30 I Ethanol hergestellt wurden, wobei die Extraktion während mindestens 4, vorzugsweise während mindestens 6 Monaten bei Umgebungstemperatur erfolgte. Die Umgebungstemperatur liegt gewöhnlich nicht unter 5 _{°} C und nicht über 30 _{°} C.

### Beispiel:

Es wurde ein Kombinationspräparat hergestellt, indem je 3,5 kg Blätter und Stiele des Bärlauchs resp. des Aronakrauts sowie 3,5 kg Früchte der Tollkirsche in je 25 I 70 %igen Ethanol in Glasbehältern angesetzt wurden. Die Behälter wurden verschlossen und während 6 Monaten bei ca. 15 bis 22 _{°} C stehen gelassen. Anschliessend wurde das alkoholische Extrakt von den extrahierten Pflanzen abgetrennt und im Verhältnis 70 % Aronakraut-Extrakt, 20 % Bärlauch-Extrakt und 10 % Tollkirschen-Extrakt gemischt.

Das derart hergestellte Kombinationspräparat zeigte gute Heilwirkung bei Hautkrebs, wobei täglich 10 ml des Präparats während einem Zeitraum von ca. 6 Wochen angewendet worden waren.

Bevorzugt werden die einzelnen oder kombinierten Extrakte direkt verwendet. Zur Vereinfachung der Verabreichung können diese jedoch auch in bekannte pharmazeutische Verabreichungsformen wie Kapseln eingearbeitet werden. Allenfalls können die extrahierten Wirkstoffe auch aus dem Extrakt abgetrennt und beispielsweise zu Tabletten, Salben etc. verarbeitet werden.

## Patentansprüche

1. Pflanzliches Heilmittel, dadurch gekennzeichnet, dass es mindestens ein alkoholisches Extrakt mindestens einer Pflanze, ausgewählt aus den Familien der Solanaceae, Araceae und Liliaceae enthält oder daraus besteht.

2. Pflanzliches Heilmittel gemäss Anspruch 1, dadurch gekennzeichnet, dass die mindestens eine Solonaceae Tollkirsche und/oder die mindestens eine Araceae Atonstab und/oder die mindestens eine Liliaceae ein Allium, insbesondere Bärlauch, ist.

3. Pflanzliches Heilmittel gemäss Anspruch 1 oder 2, dadurch gekennzeichnet, dass es Extrakte von Pflanzen aller drei genannten Familien enthält oder daraus besteht.

4. Verfahren zur Herstellung eines pflanzlichen Heilmittels gemäss einem der Ansprüche 1 bis 3, dadurch gekennzeichnet, dass von den Pflanzen aus den Familien der Liliaceae und Araceae die über dem Boden wachsenden Blätter und Stiele und von den Pflanzen aus der Familie der Solanaceae die Früchte mit Alkohol extrahiert werden.

5. Verfahren gemäss Anspruch 4, dadurch gekennzeichnet, dass identisch hergestellte Extrakte im Verhältnis von 65-75 %, insbesondere 70 % Extrakt der Aronaceae, 15-25 %, insbesondere 20 % Extrakt der Liliaceae und 5-15 %, insbesondere 10 % Extrakt der Solanaceae gemischt werden, wobei die Komponenten zusammen 100 % ergeben.

6. Verfahren gemäss Anspruch 4 oder 5, dadurch gekennzeichnet, dass 4-5 kg, insbesondere 3,5 kg der Blätter und Stiele resp. der Früchte mit 20-30 I, insbesondere 25 I Alkohol extrahiert werden.

7. Verfahren gemäss einem der Ansprüche 4 bis 6, dadurch gekennzeichnet, dass der Alkohol Ethanol von mindestens 40 Vol.-%, bevorzugt mind. 70 Vol.-% ist.

8. Verfahren gemäss einem der Ansprüche 4 bis 7, dadurch gekennzeichnet, dass die Extraktion bei Umgebungstemperatur während ca. 1/2 Jahr durchgeführt wird.

9. Verfahren gemäss einem der Ansprüche 4 bis 8, dadurch gekennzeichnet, dass die Pflanzenextrakte separat hergestellt und die Extrakte erst nach erfolgter Extraktion zusammengemischt werden.
